# EUROPÄISCHE PATENTSCHRIFT

(11) **EP 2 788 299 B1**
(45) Veröffentlichungstag und Bekanntmachung des Hinweises auf die Patenterteilung: **12.06.2024**
(21) Anmeldenummer: 12797913.6
(22) Anmeldetag: 06.12.2012
(51) Int. Cl.: C04B 24/02, C04B 28/02, C04B 40/00, C04B 103/52

(54) **STABILISIERUNG VON ROHPOLYOLEN AUS BIOMASSE**
STABILIZATION OF CRUDE POLYOLS FROM BIOMASS
STABILISATION DE POLYOLS BRUTS OBTENUS À PARTIR DE BIOMASSE

(30) Priorität: 06.12.2011 EP 11192123
(43) Veröffentlichungstag der Anmeldung: 15.10.2014
(73) Patentinhaber: Sika Technology AG, 6340 Baar (CH)
(72) Erfinder: HONERT, Dieter, 69234 Dielheim (DE); KNAUBER, Hans, 69214 Eppelheim (DE); MÜLLER, Thomas, 69115 Heidelberg (DE)
(74) Vertreter: Sika Patent Attorneys
(86) Internationale Anmeldenummer: PCT/EP2012/074640
(87) Internationale Veröffentlichungsnummer: WO 2013/083692

(56) Entgegenhaltungen:
- WO-A1-01/12581
- WO-A1-2010/062484
- WO-A2-2006/051574
- WO-A2-2006/132762
- CN-A- 101 125 742

## Beschreibung

### Technisches Gebiet

Die Erfindung betrifft die Verwendung von aus Biomasse hergestelltem stabilisiertem Rohpolyol als Mahlhilfsmittel. Des Weiteren bezieht sich die Erfindung auf eine Mahlhilfsmittelzusammensetzung umfassend besagtes Rohpolyol sowie ein Verfahren zum Mahlen eines Feststoffs.

### Stand der Technik

Ein zentraler Schritt und wesentlicher Kostenfaktor bei der Herstellung von mineralischen Bindemitteln, insbesondere Zement, ist das Mahlen der grobkörnigen mineralischen Komponenten zu feinem Pulver. So werden bei der Zementherstellung beispielsweise Klinker und, abhängig vom herzustellenden Zementtyp gegebenenfalls auch Zusatzstoffe, wie z.B. Hüttensand oder Kalkstein, zu feinen Pulver vermahlen. Zement und Zusatzstoffe können dabei grundsätzlich zusammen oder auch getrennt gemahlen werden.

Die Feinheit des mineralischen Bindemittels oder der Zusatzstoffe ist dabei ein wichtiges Qualitätsmerkmal. Beispielsweise weisen ausgehärtete Mörtel- oder Betonmischungen mit fein gemahlenen mineralischen Bindemitteln im Allgemeinen höhere Druckfestigkeiten auf als entsprechende Mischungen auf der Basis von gröber gemahlenen mineralischen Bindemitteln.

Um das Zerkleinern von mineralischen Bindemitteln oder Zusatzstoffen in Mühlen zu erleichtern und die Agglomeration der resultierenden pulverförmigen Teilchen zu verhindern, können sogenannte Mahlhilfsmittel eingesetzt werden. Sie bewirken eine starke Reduktion der Mahldauer und des zur Mahlung erforderlichen Energieaufwandes. Mahlhilfsmittel werden in Mengen von bis zu ca. 0.2%, in Ausnahmefällen bis 0.5%, bezogen auf das Mahlgut, zusammen mit diesem der Zementmühle zugegeben. Dadurch kann bei gleicher Feinheit bzw. identischem Blainewert des Bindemittels die Mühlendurchsatzleistung um 20 bis 30%, bei manchen Anlagen sogar um bis zu 50% erhöht werden.

Als Mahlhilfsmittel haben sich seit den 60er-Jahren organische Flüssigkeiten bewährt, insbesondere Glykole und Aminoalkohole. Ebenfalls bekannt ist die Verwendung von Glycerin als Mahlhilfsmittel.

Die WO 2006/132762 A2 (W.R. Grace & Co.-Conn.) offenbart in diesem Zusammenhang beispielsweise die Verwendung von Polyolen aus Biomasse. Dabei wird z.B. Rohglycerin, welches unter anderem als Nebenprodukt bei der Herstellung von Bio-Diesel entsteht, in Mahlhilfsmittelzusammensetzungen eingesetzt. Im Vergleich mit Glycerin aus fossilen Quellen kann die Mahleffizienz mit Biomasse-basierten Polyolen verbessert werden. Zudem ist Glycerin aus Biomasse in ökologischer Hinsicht vorteilhaft.

Bei der Verwendung von Polyolen aus Biomasse, insbesondere Rohglycerin, als Bestandteil von Mahlhilfsmittelzusammensetzungen, kommt es jedoch häufig zu Verschmutzungen von Pumpen und Leitungen sowie zu Störungen beim Mahlen von Feststoffen oder Bindemitteln. Weiterhin muss auch mit Verharzungseffekten gerechnet werden.

### Darstellung der Erfindung

Aufgabe der vorliegenden Erfindung ist es daher, Rohpolyole aus Biomasse, insbesondere Rohglycerin, zugänglich zu machen, welche die vorstehend genannten Nachteile weniger oder gar nicht aufweisen. Die Rohpolyole sollen insbesondere als Mahlhilfsmittel oder in Mahlhilfsmittelzusammensetzungen einsetzbar sein und den Mahlprozess von anorganischen und/oder mineralischen Feststoffen, insbesondere mineralischen Bindemitteln, erleichtern oder verbessern. Weiter soll eine entsprechende Zusammensetzung bereit gestellt werden, welche in vorteilhafter Weise als Mahlhilfsmittel einsatzbar ist.

Die Aufgaben werden erfindungsgemäss durch die Merkmale der unabhängigen Ansprüche gelöst.

Der Kern der Erfindung liegt demnach in der Verwendung eines Lösevermittlers zur Stabilisierung von aus Biomasse hergestelltem Rohpolyol.

Wie sich gezeigt hat, enthalten Rohpolyole aus Biomasse aufgrund der Herstellungsprozesse ölige Komponenten als Nebenbestandteile. Zum Beispiel enthält Rohglyzerin aus der Herstellung von Bio-Diesel typischerweise einen Anteil von Rapsöl bzw. Rapsölmethylester. Diese öligen Nebenbestandteile sind in den Rohpolyolen, z.B. in Rohglyzerin, oder in wässrigen Abmischungen der Rohpolyole wenig löslich und neigen zur Separation. Daher bilden sich bereits nach kurzer Zeit unerwünschte Trennphasen auf der Oberfläche der Rohpolyole oder des Rohglycerins, was unter anderem zu den eingangs erwähnten Nachteilen führt.

Mit Hilfe der erfindungsgemäss verwendeten Lösevermittler, insbesondere einer alkalischen Substanz, ist es jedoch möglich, eine Stabilisierung der Rohpolyole aus Biomasse zu erreichen. Es hat sich gezeigt, dass die Rohpolyole aus Biomasse aufgrund des Lösevermittlers langzeitig stabil bleiben und eine unerwünschte Phasenseparation stark reduziert oder gar vollständig unterbunden werden kann. Die Rohpolyole aus Biomasse liegen somit in Form einer stabilen und im Wesentlichen homogenen Phase vor.

Bei der Verwendung der so behandelten Rohpolyole aus Biomasse als Mahlhilfsmittel können Verschmutzungen von Pumpen und Leitungen sowie Störungen beim Mahlen von Feststoffen oder Bindemitteln stark reduziert werden. Auch Verharzungseffekte nehmen signifikant ab.

Zudem wurde festgestellt, dass die verwendeten Lösevermittler den Mahlvorgang an sich oder die Eigenschaften des Mahlguts kaum oder gar nicht negativ beeinträchtigt.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Ein erster Aspekt der vorliegenden Erfindung betrifft die Verwendung eines Rohpolyols gemäß Anspruch 1.

Der Lösevermittler ist dabei insbesondere eine Substanz, welche mit Glyzerin (1,2,3-Propantriol) mischbar, im Besonderen homogen mischbar, ist und mit Vorteil eine Tendenz zur Phasenphasenseparation in Rohpolyol reduziert. Bevorzugt sind wenigstens 5 Gew.-%, insbesondere wenigstens 10 Gew.-%, des Lösevermittlers bei Raumtemperatur mit Glycerin mischbar.

"Biomasse" oder "nachwachsende Rohstoffe" umfasst vorliegend lebende als auch tote Materie. Dies können beispielsweise Pflanzen, Tiere, Mikroorganismen, Holz, Laub oder Stroh sein. Fossile Energieträger, wie z.B. Kohle, Erdöl oder Erdgas, welche in geologischer Vorzeit aus Biomasse entstanden sind, werden hingegen nicht der Biomasse zugeordnet.

Polyole aus fossilen Quellen und Polyole aus Biomasse lassen sich aufgrund ihres unterschiedlichen Anteils an ¹⁴C-Isotopen durch Radiokohlenstoffdatierung unterscheiden. Entsprechende Verfahren, wie z.B. die Zählrohrmethode nach Libby, die Flüssigszintillationsspektrometrie, oder der Beschleunigermassenspektrometrische Nachweis, sind dem Fachmann an sich bekannt.

Ein "Polyol" steht insbesondere für eine organische Verbindung mit wenigstens 2 Hydroxygruppen. Bevorzugt sind Verbindungen mit genau 2 oder 3 Hydroxygruppen. Beispiele sind Ethylenglycol (1,2-Ethandiol) oder Glycerin (1,2,3-Propantriol).

Ein "aus Biomasse hergestelltes Polyol" kann vorliegend insbesondere als Zusammensetzung umfassend wenigstens ein Polyol sowie einen Anteil an weiteren organischen und/oder anorganischen Substanzen verstanden werden. Die weiteren Substanzen unterscheiden sich dabei chemisch vom wenigstens einen Polyol. Beispielsweise können Fettsäuren, Fettsäureester, Alkohole, Wasser und/oder Zucker als weitere Substanzen vorliegen. Ein Anteil der weiteren Substanzen beträgt vorzugsweise weniger als 50 Gew.-%, insbesondere weniger als 20 Gew.-%, noch weiter bevorzugt weniger als 15 Gew.-%. Insbesondere liegt ein Anteil der weiteren Substanzen bei 0.005 - 30 Gew.-%, bevorzugt 5 - 20 Gew.-% oder 5 - 10 Gew.-%.

"Stabilisierung" meint vorliegend insbesondere eine Phasenstabilisierung. Die Phase der Polyole aus Biomasse soll dabei über die Zeit möglichst stabil bleiben. Anders ausgedrückt sollen die Zusammensetzung und die Eigenschaften der Polyolphase stabilisiert oder aufrechterhalten werden. Insbesondere soll eine Phasenseparation oder eine Abtrennung einer weiteren Phase aus der Polyolphase reduziert oder unterbunden werden.

Unter dem Begriff "Mahlen" oder "Mahlprozess" wird insbesondere ein Verfahren verstanden, bei welchem eine mittlere Korngrösse eines Feststoffs oder eines Gemischs von verschiedenen Feststoffen reduziert wird. Dies erfolgt beispielsweise in einer Mühle beim Mahlen von Klinker, gegebenenfalls zusammen mit inerten und/oder aktiven Zusatzstoffen, wie z.B. mit Gips, Anhydrit, α-Halbhydrat, β-Halbhydrat, latenthydraulischen Bindemitteln, puzzolanischen Bindemittel und/oder inerten Füllstoffen. Die genannten Feststoffe können auch einzeln gemahlen werden. Typischerweise wird der Feststoff oder das Gemisch von verschiedenen Feststoffen, insbesondere ein mineralisches Bindemittel, beim Mahlen auf einen Blaine-Wert von wenigstens 500 cm²/g, insbesondere wenigstens 1'000 cm²/g, bevorzugt wenigstens 2'000 cm²/g, noch weiter bevorzugt wenigstens 2'500 cm²/g, gemahlen.

Ein "Feststoff" ist im vorliegenden Zusammenhang insbesondere ein anorganischer und/oder mineralischer Feststoff. Insbesondere ist der Feststoff ein anorganischer Stoff für Bauzwecke, beispielsweise als Bestandteil für Zement-, Mörtel- und/oder Betonzusammensetzungen. Bevorzugt ist der Feststoff ein mineralisches Bindemittel und/oder ein Zusatzstoff für ein mineralisches Bindemittel. Der Feststoff kann grundsätzlich in grober Form, z.B. als (ungemahlener) Klinker, und/oder bereits teilweise vermahlen vorliegen.

Ein "mineralisches Bindemittel" ist dabei im Besonderen ein Bindemittel, insbesondere ein anorganisches Bindemittel, welches in Anwesenheit von Wasser in einer Hydratationsreaktion zu festen Hydraten oder Hydratphasen reagiert. Dies kann beispielsweise ein hydraulisches Bindemittel (z.B. Zement oder hydraulischer Kalk), ein latent hydraulisches Bindemittel (z.B. Schlacke oder Hüttensand), ein puzzolanisches Bindemittel (z.B. Flugasche, Trass oder Reisschalenasche) und/oder ein nicht hydraulisches Bindemittel (Gips oder Weisskalk) sein. Auch Mischungen der verschiedenen Bindemittel sind möglich.

Als Polyol aus Biomasse bevorzugt ist Rohglycerin. Mit Vorteil weist das Rohglycerin einen Gehalt an reinem Glycerin oder 1,2,3-Propantriol von wenigstens 20 Gew.-%, bevorzugt wenigstens 50 Gew.-%, insbesondere wenigstens 75 Gew.-%, weiter bevorzugt wenigstens 80 Gew.-%, auf. Insbesondere liegt ein Anteil von Glycerin bei 20 - 98 Gew.-%, insbesondere 50 - 95 Gew.-%, speziell 50 - 90 Gew.-%.

Weitere organische und/oder anorganische Substanzen verfügen zusammen mit Vorteil über einen Anteil von weniger als 50 Gew.-%, insbesondere 0.005 - 30 Gew.-%, bevorzugt 5 - 20 Gew.-%. Ein Anteil an anorganischen Alkalimetallsalzen liegt insbesondere bei weniger als 20 Gew.-%, insbesondere 1 - 15 Gew.-%, bevorzugt 1 - 10 oder 2 - 8 Gew.-%. Bei den Salzen handelt es sich insbesondere um Natriumchlorid, Natriumsulfid, Kaliumchlorid, Kaliumsulfat oder Mischungen davon. Speziell sind dies Natriumchlorid und/oder Natriumsulfate. Im Besonderen Natriumchlorid.

Besonders geeignetes Rohglyzerin stammt z.B. aus der Biodieselproduktion. Biodiesel wird unter anderem durch Umesterung von Rapsöl oder Sojaöl mit Methanol hergestellt werden. Dabei fällt als Nebenprodukt nebst anderem auch Rohglyzerin an, welches aufgrund seiner Zusammensetzung in vorliegenden Zusammenhang vorteilhaft ist. Zudem ist Rohglyzerin aus der Biodieselproduktion weltweit in grossen Mengen verfügbar.

Das Rohpolyol ist bevorzugt ein Rohglyzerin mit einem wie vorstehend beschriebenen Gehalt an reinem Glycerin oder 1,2,3-Propantriol sowie einem wie vorstehend beschriebenen Anteil an weiteren organischen und/oder anorganischen Substanzen. Das Rohglyzerin oder die weiteren organischen und/oder anorganischen Substanzen umfassen insbesondere ölige Komponenten als Nebenbestandteile, im Besonderen Öle, Fettsäuren und/oder Fettsäureester, speziell Rapsöl und/oder Rapsölmethylester.

Das Rohglyzerin umfasst ölige Komponenten als Nebenbestandteile, im Besonderen Öle, Fettsäuren und/oder Fettsäureester, welche sich bei der Herstellung von Rohglyzerin in der Biodieselproduktion oder bei der Herstellung von Rohglyzerin durch Umesterung von pflanzlichen Ölen und/oder Fetten, insbesondere Rapsöl und/oder Sojaöl, mit Alkoholen, bevorzugt Methanol, ergeben.

Insbesondere enthält das Rohglyzerin die öligen Nebenbestandteile, im Besonderen Fettsäuren und/oder Fettsäureester, mit einem Anteil von weniger als 50 Gew.-%, insbesondere 0.005 - 30 Gew.-%, bevorzugt 5 - 20 Gew.-% oder 5 - 10 Gew.-%.

Es kann aber grundsätzlich auch Rohglyzerin aus anderen Quellen eingesetzt werden. So fällt Rohglyzerin z.B. auch bei der Seifenherstellung aus Kokosfett, Olivenöl, Palmöl und tierischen Fetten wie Talg, Schmalz oder Fett aus Knochen an.

Der Lösevermittler umfasst eine alkalische Substanz oder er besteht daraus. Der Ausdruck "alkalische Substanz" steht im vorliegenden Zusammenhang insbesondere für eine Substanz, welche, falls einer wässrigen Lösung zugegeben, deren pH-Wert anzuheben vermag. Die alkalische Substanz ist zudem insbesondere wie vorstehend beschrieben mit Glycerin mischbar.

Erfindungsgemäß umfasst die alkalische Substanz insbesondere ein Alkanolamin, ein Fettamin und/oder ein Alkalihydroxid. Insbesondere besteht die alkalische Substanz aus einem der genannten Vertreter oder aus Mischungen davon.

Geeignete Alkanolamine sind z.B. Triethanolamin, Diethanolamin, Ethanolamin, Diisopropanolamin, Triisopropanolamin, Diethanol-isopropanolamin, Ethanol-diisopropanolamin, N-Methyl-diisopropanolamin, N,N,N',N'-Tetrakis(2-hydroxyethyl)ethylendiamin, N,N,N',N'-Tetrakis(2-hydroxypropyl)ethylenediamin, N-Methyldiethanolamin, N,N-Dimethylethanolamin, N,N-Diethylethanolamin. Insbesondere geeignet sind Diethanolamin, Triisopropanolamin, Diisopropanolamin, N-Methyl-diisopropanolamin. Besonders bevorzugt sind Triethanolamin und/oder Triisopropanolamin.

Als Fettamine könne z.B. Octylamin, Dodecylamin, Tetradecylamin, Kokusnussfettamin, Soyabohnenfettamin und/oder Stearylamin eingesetzt werden.

Vorteilhafte Alkalihydroxide sind beispielsweise Natriumhydroxid, und/oder Kaliumhydroxid.

Besonders bevorzugt ist die alkalische Substanz ein Alkanolamin. Dies ist überraschend, da derartige Substanzen im Allgemeinen keine typischen Lösevermittler darstellen. Triethanolamin und/oder Triisopropanolamin sind dabei besonders vorteilhaft. Speziell Triethanolamin. Derartige Substanzen ermöglichen einerseits eine gute Stabilisierung des Rohglyzerins, andererseits sind dies Stoffe im Hinblick auf die Verwendung des Rohgylzerins in Mahlhilfsmittelzusammensetzungen vorteilhaft. Dies weil Alkanolamine unter anderem auch als Mahlhilfsmittel wirken können und mit den üblicherweise in Mahlhilfsmittelzusammensetzungen vorliegenden Substanzen relativ gut kompatibel sind.

Beschrieben ist auch ein Lösevermittler, der ein Glykol und/oder einen Glykolether umfasst.

Als Glykole kommen beispielsweise Ethylenglykol, Diethylenglykol, Triethylenglykol, Tetraethylenglykol, Propylenglykol, Dipropylenglykol, und/oder Tripropylenglykol in Frage.

Mögliche Glykolether sind z.B. Butylglykol, n-Hexylglykol, Methyldiglykol , Ethyldiglykol, Butyldiglykol, Butyltriglykol, Monophenylglykol, Methoxypropanol, Ethoxypropanol, Dipropylenglykolmethylether, Tripropylenglykolmethylether, Propylenglykol-n-butylether, Dipropylenglykol-n-butylether, Tripropylenglykol-n-butylether und/oder Hexylenglykol.

Insbesondere geeignet sind Monohydroxyglykolether. Als besonders bevorzugt hat sich Butyldiglykol erwiesen. Butyldiglykol wird auch 2-(2-Butoxyethoxy)ethanol) genannt. Mit Butyldiglykol konnte eine besonders effektive Stabilisierung von Rohpolyol beaobachtet werden.Wie es sich gezeigt hat, wird der Lösevermittler mit einem Anteil von 1 - 9 Gew.-%, weiter bevorzugt 5 - 9 Gew.-eingesetzt.

Die Gewichtanteile sind jeweils bezogen auf das Gesamtgewicht der Zusammensetzung enthaltend wenigstens ein aus Biomasse hergestelltes Polyol.

Erfindungsgemäß ist das Rohpolyol Bestandteil einer Mahlhilfsmittelzusammensetzung. Mit anderen Worten kann das durch Verwendung eines Lösevermittlers, einer alkalischen Substanz stabilisierte Rohpolyol als Bestandteil einer Mahlhilfsmittelzusammensetzung zum Mahlen von Feststoffen, insbesondere mineralischen Bindemitteln und/oder von Zusatzstoffen für mineralische Bindemittel, eingesetzt werden.

Die Mahlhilfsmittelzusammensetzung enthält dabei zusätzlich wenigstens ein Additiv, ausgewählt aus der Gruppe bestehend aus Polycarboxylaten, Glykolen, Aminen, organische Säuren, Ligninsulfonaten, Luftporenbildnern, Entschäumern und/oder Verzögerern. Durch derartige Additive oder Mahlhilfsmittelrohstoffe lassen sich die Mahlhilfsmittelzusammensetzungen gezielt an spezifische Anforderungen anpassen. Dies insbesondere in Verbindung Lösevermittlern in Form von alkalischen Substanzen, speziell Alkanolaminen.

Besonders bevorzugt sind Mahlhilfsmittelzusammensetzungen, welche nebst dem Rohpolyol wenigstens ein Polycarboxylat enthalten. Wie in der WO 2005/123621 A1 beschrieben, wirken derartige Polymere selbst als Mahlhilfsmittel. Durch Kombination mit Rohpolyol kann eine ökonomische Anpassung der Mahlhilfsmittelzusammensetzung an spezifische Erfordernisse erreicht werden.

Das wenigstens eine Polycarboxylat ist insbesondere ein Kammpolymer umfassend ein Polycarboxylatrückgrat mit daran gebundenen Polyetherseitenketten. Die Seitenketten sind dabei insbesondere über Ester-, Ether- und/oder Amidgruppen an das Polycarboxylatrückgrat gebunden.

Entsprechende Polycarboxylatether und Herstellungsverfahren sind beispielsweise offenbart in EP 1 138 697 B1 auf Seite 7 Zeile 20 bis Seite 8 Zeile 50, sowie in dessen Beispielen oder in EP 1 061 089 B1 auf Seite 4, Zeile 54 bis Seite 5 Zeile 38 sowie in deren Beispielen. In einer Abart davon, wie sie in EP 1 348 729 A1 auf Seite 3 bis Seite 5 sowie in deren Beispielen beschrieben sind, kann das Kammpolymer in festem Aggregatszustand hergestellt werden.

Derartige Kammpolymere werden auch von Sika Schweiz AG unter der Handelsnamenreihe ViscoCrete^{®} kommerziell vertrieben.

Ein weiterer Aspekt der Erfindung betrifft eine Mahlhilfsmittelzusammensetzung gemäß Anspruch 8.

Der Lösevermittler liegt in einer Menge von 1 - 9 Gew.-%, bevorzugt 2 - 7 Gew.-%, bezogen auf das Gesamtgewicht der Mahlhilfsmittelzusammensetzung, vor.

Weiter kann die Zusammensetzung insbesondere die bereits vorstehend genannten Additive enthalten.

Besonders bevorzugt ist eine Mahlhilfsmittelzusammensetzung enthaltend oder bestehend aus:
a) 10 - 80 Gew.-%, insbesondere 20 - 50 Gew.-%, Rohpolyol in Form von Rohglyzerin
b) 0.1 - 9 Gew.-%, insbesondere 1 - 7 Gew.-%, der alkalischen Substanz, bevorzugt ein Alkanolamin
c) 0 - 40 Gew.-% eines Polycarboxylats
d) 0 - 60 Gew.-% eines Glykols
e) 0 - 10 Gew.-% einer organischen Säure
f) gegebenenfalls Rest auf 100 Gew.-% Wasser. Speziell vorteilhaft sind dabei Zusammensetzung enthaltend 5 - 40 Gew.-%, insbesondere 10- 30 Gew.-%, des Polycarboxylats.

Glykol weist insbesondere einen Anteil von 5 - 50 Gew.-%, insbesondere 25 - 40 Gew.-%, auf.

Ein Anteil der organischen Säure von 1 - 10 Gew.-%, insbesondere 2 - 6 Gew.-%, hat sich als optimal erwiesen.

Ein weiterer Aspekt der Erfindung betrifft eine Bindemittelzusammensetzung enthaltend ein mineralisches Bindemittel sowie eine Mahlhilfsmittelzusammensetzung. Das mineralische Bindemittel und die Mahlhilfsmittelzusammensetzung liegen dabei insbesondere wie vorstehend beschreiben vor. Die Mahlhilfsmittelzusammensetzung weist insbesondere einen Anteil von 0.001 - 1 Gew.-% bezogen auf das mineralische Bindemittel auf.

Weiter bezieht sich die Erfindung auf ein Verfahren zum Mahlen eines Feststoffs, insbesondere eines mineralisches Bindemittels, wobei der Feststoff mit einer Mahlhilfsmittelzusammensetzung gemahlen wird. Die Mahlhilfsmittelzusammensetzung wird dabei insbesondere mit einem Anteil von 0.001 - 1.0 Gew.-%, bezogen auf den zu mahlenden Feststoff eingesetzt.

Aus der nachfolgenden Detailbeschreibung und der Gesamtheit der Patentansprüche ergeben sich weitere vorteilhafte Ausführungsformen und Merkmalskombinationen der Erfindung.

### Ausführungsbeispiele

### 1. Stabilisierung von Rohglyzerin

In einem Gefäss wurde Rohglyzerin (Wetterauer Agrarservice GmbH - Glyzeringehalt: 80%) aus der Biodieselproduktion mit den in Tabelle 1 angegebenen Mengen an alkalischen Substanzen versetzt und während 5 min gerührt. Anschliessend wurden die Proben stehen gelassen und die Zeitdauer (Separationszeit) bis zur Bildung einer sichtbaren Trennphase bestimmt. Tabelle 1 gibt einen Überblick über die hergestellten Proben und deren Eigenschaften.

**Tabelle 1**

| **Probe** | **Alkalische Substanz (Anteil in Gew.-%)** | **Separationszeit** |
|---|---|---|
| **A** | Triethanolamin (5.0 %) | > 6 Monate |
| **B*** | Triethanolamin (15 %) | > 6 Monate |
| **C*** | Triethanolamin (50 %) | > 6 Monate |
| **D*** | Triisopropanolamin (15 %) | > 6 Monate |
| **E*** | Butyldiglykol (15 %) | > 6 Monate |
| **V*** | Ohne | 1 Woche |

| | | |
|---|---|---|
| * nicht gemäss vorliegender Erfindung | | |

Aus Tabelle 1 ist ersichtlich, dass alkalische Substanzen das Rohglyzerin dauerhaft zu stabilisieren vermögen. Die Proben **A** - **E** zeigen auch nach 6 Monaten keine sichtbare Phasenseparation. Dies im Gegensatz zur nicht stabilisierten Vergleichsprobe **V,** auf welcher sich bereits nach 4 Wochen eine Trennschicht bildete.

### 2. Mahlversuche

Die Proben **A - D** wurden als Additive beim Mahlen von Zementklinker als Mahlhilfsmittel auf einer Laborkugelmühle eingesetzt. Dabei wurden jeweils 300 g eines Zementklinkers (Vigier) unter identischen Bedingungen mit 0.02 Gew.-% des jeweiligen Additivs (Menge an Probe **A** - **D** bezogen auf Zementklinker) auf Laborkugelmühlen gemahlen. Versuch **M0** ist eine Referenzprobe ohne Additiv. Die Bedingungen, wie z.B. die Mahldauer, wurden bei allen Mahlversuchen konstant gehalten.

Nach erfolgtem Mahlvorgang wurde die Feinheit nach Blaine analog Norm EN 196-6 sowie der Siebrückstand der Partikel über 32 µm (in Gew.-% bezogen auf sämtliche Partikel) analog Norm EN 196-6 (Mai 2010) mit einem 32 µm Sieb bestimmt. Die Resultate wurden zudem durch Lasergranulometrie verifiziert.

Tabelle 3 gibt einen Überblick über die durchgeführten Mahlversuche und die entsprechenden Resultate.

**Tabelle 3**

| **Versuch** | **M0*** | **M1** | **M2*** | **M3*** | **M4*** |
|---|---|---|---|---|---|
| Additiv | - | **A** | **B** | **C** | **D** |
| Feinheit [cm²/g] | 1745 | 2215 | 2275 | 2460 | 2315 |
| Siebrückstand > 32 µm [Gew.-%] | 45.3 | 38.1 | 36.2 | 33.1 | 35.8 |

| | | | | | |
|---|---|---|---|---|---|
| * nicht gemäss vorliegender Erfindung | | | | | |

Aus Tabelle 3 ist ersichtlich, dass stabilisiertes Rohglyzerin in vorteilhafter Weise als Mahlhilfsmittel eingesetzt werden kann. Verstopfungen oder Verschmutzungen von Pumpen und Leitungen sowie Störungen beim Mahlen oder Verharzungen wurden keine beobachtet.

Die vorstehend beschriebenen Ausführungsformen sind jedoch lediglich als illustrative Beispiele zu verstehen, welche im Rahmen der Erfindung beliebig abgewandelt werden können.

**Tabelle 3**

| **Versuch** | **M0** | **M1** | **M2** | **M3** | **M4** | **M5** |
|---|---|---|---|---|---|---|
| Additiv | - | **A** | **B** | **C** | **D** | **MZ** |
| Feinheit [cm²/g] | 1745 | 2215 | 2275 | 2460 | 2315 | 2550 |
| Siebrückstand > 32 µm [Gew.-%] | 45.3 | 38.1 | 36.2 | 33.1 | 35.8 | 32.2 |

Aus Tabelle 3 ist ersichtlich, dass stabilisiertes Rohglyzerin als auch die darauf basierende Mahlhilfsmittelzusammensetzung **MZ** in vorteilhafter Weise als Mahlhilfsmittel eingesetzt werden können. Verstopfungen oder Verschmutzungen von Pumpen und Leitungen sowie Störungen beim Mahlen oder Verharzungen wurden keine beobachtet.

Die vorstehend beschriebenen Ausführungsformen sind jedoch lediglich als illustrative Beispiele zu verstehen, welche im Rahmen der Erfindung beliebig abgewandelt werden können.

## Patentansprüche

1. Verwendung von aus Biomasse hergestelltem Rohpolyol als Mahlhilfsmittel zum Mahlen von Feststoffen, wobei das Rohpolyol ölige Komponenten als Nebenbestandteile enthält und durch einen Lösevermittler stabilisiert ist, und wobei der Lösevermittler eine alkalische Substanz umfasst oder daraus besteht, wobei die alkalische Substanz insbesondere ein Alkanolamin, ein Fettamin und/oder ein Alkalihydroxid umfasst oder daraus besteht, und wobei der Lösevermittler mit einem Anteil von 1 - 9 Gew.-%, bezogen auf das Gesamtgewicht der Zusammensetzung enthaltend wenigstens ein aus Biomasse hergestelltes Polyol, eingesetzt wird.

2. Verwendung nach Anspruch 1, **dadurch gekennzeichnet, dass** das Rohpolyol einen Anteil an reinem Polyol oder an 1,2,3-Propantriol von 20 - 95 Gew.-%, speziell 50 - 90 Gew.-%, aufweist.

3. Verwendung nach wenigstens einem der vorangehenden Ansprüche, **dadurch gekennzeichnet, dass** das Rohpolyol Rohglycerin ist.

4. Verwendung nach Anspruch 3, **dadurch gekennzeichnet, dass** das Rohglyzerin aus der Biodieselproduktion stammt.

5. Verwendung nach wenigstens einem der vorangehenden Ansprüche, **dadurch gekennzeichnet, dass** die alkalische Substanz ein Alkanolamin, insbesondere Triethanolamin und/oder Triisoproanolamin, ist.

6. Verwendung nach wenigstens einem der vorangehenden Ansprüche, **dadurch gekennzeichnet, dass** der Lösevermittler ein Glykol und/oder ein Glykolether, insbesondere Butyldiglykol, umfasst.

7. Verwendung nach wenigstens einem der Ansprüche 1 - 6, **dadurch gekennzeichnet, dass** das als Mahlhilfsmittel verwendete Rohpolyol zusätzlich wenigstens ein Additiv, ausgewählt aus der Gruppe bestehend aus Polycarboxylaten, Glykolen, Aminen, organische Säuren, Ligninsulfonaten, Luftporenbildnern, Entschäumern und/oder Verzögerern enthält.

8. Mahlhilfsmittelzusammensetzung umfassend ein aus Biomasse hergestelltes Rohpolyol enthaltend ölige Komponenten als Nebenbestandteile sowie einen Lösevermittler, wobei
- der Lösevermittler eine alkalische Substanz umfasst oder daraus besteht, wobei die alkalische Substanz insbesondere ein Alkanolamin, ein Fettamin und/oder ein Alkalihydroxid umfasst oder daraus besteht, und
- der Lösevermittler in einer Menge von 1 - 9 Gew.-%, insbesondere 2 -7 Gew.-%, bezogen auf das Gesamtgewicht der Zusammensetzung, vorliegt und
- wobei die Mahlhilfsmittelzusammensetzung zusätzlich wenigstens ein Additiv, ausgewählt aus der Gruppe bestehend aus Polycarboxylaten, Glykolen, Aminen, organische Säuren, Ligninsulfonaten, Luftporenbildnern, Entschäumern und/oder Verzögerern enthält.

9. Zusammensetzung nach Anspruch 8, enthaltend oder bestehend aus:
a) 10 - 80 Gew.-%, insbesondere 20 - 50 Gew.-%, Rohpolyol in Form von Rohglyzerin,
b) 0.1 - 9 Gew.-%, insbesondere 1 - 7 Gew.-%, der alkalischen Substanz, bevorzugt ein Alkanolamin,
c) 0 - 40 Gew.-% eines Polycarboxylats,
d) 0 - 60 Gew.-% eines Glykols,
e) 0 - 10 Gew.-% einer organischen Säure,
f) gegebenenfalls Rest auf 100 Gew.-% Wasser.

10. Zusammensetzung nach Anspruch 9, **dadurch gekennzeichnet, dass** 5 - 40 Gew.-%, insbesondere 10 - 30 Gew.-%, des Polycarboxylats und/oder 5 - 50 Gew.-%, insbesondere 25 - 40 Gew.-%, des Glykols und/oder 1 - 10 Gew.-%, insbesondere 2 - 6 Gew.-%, der organischen Säure enthalten sind.

11. Verfahren zum Mahlen eines Feststoffs, insbesondere eines mineralischen Bindemittels, wobei der Feststoff mit einer Zusammensetzung nach einem der Ansprüche 8 - 10 gemahlen wird.

## Claims

1. Use of crude polyol produced from biomass as grinding aid for grinding solids, wherein the crude polyol comprises oily components as secondary constituents and is stabilized by a solubilizer, and wherein the solubilizer comprises an alkaline substance or consists thereof, wherein the alkaline substance comprises in particular an alkanolamine, a fatty amine and/or an alkali metal hydroxide or consists thereof, and wherein the solubilizer is employed in a content of 1-9% by weight based on the total weight of the composition comprising at least one polyol produced from biomass.

2. Use according to Claim 1, **characterized in that** the crude polyol has a content of pure polyol or of propane-1,2,3-triol of 20-95% by weight, especially 50-90% by weight.

3. Use according to at least one of the preceding claims, **characterized in that** the crude polyol is crude glycerol.

4. Use according to Claim 3, **characterized in that** the crude glycerol originates from biodiesel production.

5. Use according to at least one of the preceding claims, **characterized in that** the alkaline substance is an alkanolamine, in particular triethanolamine and/or triisopropanolamine.

6. Use according to at least one of the preceding claims, **characterized in that** the solubilizer comprises a glycol and/or a glycol ether, in particular butyl diglycol.

7. Use according to at least one of Claims 1-6, **characterized in that** the crude polyol used as grinding aid additionally comprises at least one additive selected from the group consisting of polycarboxylates, glycols, amines, organic acids, lignin sulfonates, air pore formers, defoamers and/or other retarders.

8. Grinding aid composition including a crude polyol produced from biomass, comprising oily components as secondary constituents and also a solubilizer, wherein
- the solubilizer comprises an alkaline substance or consists thereof, wherein the alkaline substance comprises in particular an alkanolamine, a fatty amine and/or an alkali metal hydroxide or consists thereof, and
- the solubilizer is present in an amount of 1-9% by weight, in particular 2-7% by weight, based on the total weight of the composition and
- wherein the grinding aid composition additionally comprises at least one additive selected from the group consisting of polycarboxylates, glycols, amines, organic acids, lignin sulfonates, air pore formers, defoamers and/or other retarders.

9. Composition according to Claim 8, comprising or consisting of:
a) 10-80% by weight, in particular 20-50% by weight, of crude polyol in the form of crude glycerol,
b) 0.1-9% by weight, in particular 1-7% by weight, of the alkaline substance, preferably an alkanolamine,
c) 0-40% by weight of a polycarboxylate,
d) 0-60% by weight of a glycol,
e) 0-10% by weight of an organic acid,
f) optionally the remainder to 100% of water.

10. Composition according to Claim 9, **characterized in that** it contains 5-40% by weight, in particular 10-30% by weight, of the polycarboxylate and/or 5-50% by weight, in particular 25-40% by weight, of the glycol and/or 1-10% by weight, in particular 2-6% by weight, of the organic acid.

11. Process for grinding a solid, in particular a mineral binder, wherein the solid is ground with a composition according to any of Claims 8-10.

## Revendications

1. Utilisation de polyol brut préparé à partir de biomasse en tant qu'adjuvant de broyage pour le broyage de solides, le polyol brut contenant des composants huileux en tant que constituants secondaires et étant stabilisé par un promoteur de solubilisation et le promoteur de solubilisation comprenant ou étant constitué par une substance alcaline, la substance alcaline comprenant ou étant constituée par, en particulier, une alcanolamine, une amine grasse et/ou un hydroxyde de métal alcalin et le promoteur de solubilisation étant utilisé en une proportion de 1-9% en poids, par rapport au poids total de la composition contenant au moins un polyol préparé à partir de biomasse.

2. Utilisation selon la revendication 1, **caractérisée en ce que** le polyol brut présente une proportion de polyol pur ou de 1,2,3-propanetriol de 20-95% en poids, en particulier de 50-90% en poids.

3. Utilisation selon au moins l'une des revendications précédentes, **caractérisée en ce que** le polyol brut est le glycérol brut.

4. Utilisation selon la revendication 3, **caractérisée en ce que** le glycérol brut provient de la production de biodiesel.

5. Utilisation selon au moins l'une des revendications précédentes, **caractérisée en ce que** la substance alcaline est une alcanolamine, en particulier la triéthanolamine et/ou la triisopropanolamine.

6. Utilisation selon au moins l'une des revendications précédentes, **caractérisée en ce que** le promoteur de solubilisation comprend un glycol et/ou un glycoléther, en particulier le butyldiglycol.

7. Utilisation selon au moins l'une des revendications 1-6, **caractérisée en ce que** le polyol brut utilisé en tant qu'adjuvant de broyage contient en outre au moins un additif, choisi dans le groupe constitué par les polycarboxylates, les glycols, les amines, les acides organiques, les sulfonates de lignine, les agents de formation de pores d'air, les antimousses et/ou les retardateurs.

8. Composition d'adjuvant de broyage comprenant un polyol brut préparé à partir de biomasse, contenant des composants huileux en tant que constituants secondaires ainsi qu'un promoteur de solubilisation,
- le promoteur de solubilisation comprenant ou étant constitué par une substance alcaline, la substance alcaline comprenant ou étant constituée par, en particulier, une alcanolamine, une amine grasse et/ou un hydroxyde de métal alcalin et
- le promoteur de solubilisation se trouvant en une quantité de 1-9% en poids, en particulier de 2-7% en poids, par rapport au poids total de la composition et
- la composition d'adjuvant de broyage contenant en outre au moins un additif, choisi dans le groupe constitué par les polycarboxylates, les glycols, les amines, les acides organiques, les sulfonates de lignine, les agents de formation de pores d'air, les antimousses et/ou les retardateurs.

9. Composition selon la revendication 8, contenant ou constituée par
a) 10-80% en poids, en particulier 20-50% en poids, de polyol brut sous forme de glycérol brut,
b) 0,1-9% en poids, en particulier 1-7% en poids de la substance alcaline, de préférence une alcanolamine,
c) 0-40% en poids d'un polycarboxylate,
d) 0-60% en poids d'un glycol,
e) 0-10% en poids d'un acide organique,
f) le cas échéant, le reste jusqu'à 100% en poids étant de l'eau.

10. Composition selon la revendication 9, **caractérisée en ce qu'**elle contient 5-40% en poids, en particulier 10-30% du polycarboxylate et/ou 5-50% en poids, en particulier 25-40% en poids du glycol et/ou 1-10% en poids, en particulier 2-6% en poids, de l'acide organique.

11. Procédé pour le broyage d'un solide, en particulier d'un liant minéral, le solide étant broyé avec une composition selon l'une des revendications 8-10.
